# EUROPÄISCHE PATENTSCHRIFT

(11) **EP 0 476 370 B1**
(45) Veröffentlichungstag und Bekanntmachung des Hinweises auf die Patenterteilung: **06.12.1995**
(21) Anmeldenummer: 91114380.8
(22) Anmeldetag: 27.08.1991
(51) Int. Cl.: C07C 67/08, C07C 67/52, C07C 69/14

(54) **Verfahren zur Abtrennung von Wasser aus einem Wasser und Alkohole und/oder Carbonsäuren und/oder Carbonsäureester enthaltenden Gemisch**
Process for the separation of water from mixtures containing water and alcohols and/or carboxylic acids and/or carboxylic acid esters
Procédé de séparation d'eau d'un mélange contenant de l'eau et des alcools et/ou des acides carboxyliques et/ou des esters d'acides carboxyliques

(30) Priorität: 15.09.1990 DE 4029349
(43) Veröffentlichungstag der Anmeldung: 25.03.1992
(73) Patentinhaber: BASF Aktiengesellschaft, 67063 Ludwigshafen (DE)
(72) Erfinder: Spiske, Luise, Dr., W-6104 Seeheim-Jugenheim (DE); Meissner, Harald, Dr., W-6702 Bad Duerkheim (DE); Hefner, Werner, Dr., W-6840 Lampertheim (DE); Huebner, Andreas, Dr., W-4750 Unna (DE); Steinhauser, Hermann, Dr., W-5000 Köln 1 (DE); Ellinghorst, Guido, Dr., W-5063 Overath (DE)

(56) Entgegenhaltungen:
- EP-A- 0 334 453
- US-A- 3 950 247

## Beschreibung

Die vorliegende Erfindung betrifft ein Verfahren zur Abtrennung von Wasser aus einem Wasser und Alkohole und/oder Carbonsäuren und/oder Carbonsäureester enthaltenden Gemisch, wie es beispielsweise als Reaktionsgemisch bei der Carbonsäureesterherstellung erhalten wird, durch Pervaporation oder Dämpfepermeation.

Aus EP-A1-0 210 055 ist bereits ein Verfahren zur Herstellung flüssiger Ester durch Umsetzung eines Alkohols mit einer Carbonsäure in Gegenwart eines sauren Katalysators bekannt, bei dem das Reaktionswasser kontinuierlich durch Pervaporation über eine Membran aus dem Reaktionsgemisch abgetrennt wird. Die bei dem bekannten Verfahren verwendeten Membranen weisen jedoch keine zufriedenstellende Permeabilität und Selektivität für Wasser auf. Eine möglichst hohe Wasserselektivität der Membranen ist jedoch erforderlich, um den Apparate- und Energiebedarf für die Verdampfung und Kondensation im Pervaporationsteil einer Anlage und die Belastung des abgetrennten Wassers mit organischen Verbindungen niedrig zu halten. Hohe Permeabilitäten der Membranen werden benötigt, damit der Bedarf an Membranfläche, der wesentlich zu den Investitions- und Instandhaltungskosten der Membrantrennanlage beiträgt, niedrig gehalten wird.

Die EP-A 0 334 453 beschreibt ein Verfahren zur Herstellung von durch Plasmapolymerisation erhaltenen Membranen (Anspruch 1). Diese Membranen wurden jedoch nur zur Trennung gasförmiger Mischungen aus Kohlendioxid und Methan, welche die gleiche Ausgangskonzentration besaßen, eingesetzt. Es findet sich kein Hinweis, daß diese Membranen zur Abtrennung von Wasser aus organischen Mischungen geeignet wären.

Es bestand daher Bedarf nach einem Verfahren zur Abtrennung von Wasser aus einem Wasser und Alkohole und/oder Carbonsäuren und/oder Carbonsäureester enthaltenden Gemisch, welches die Nachteile der bekannten Verfahren vermeidet oder vermindert.

Es wurde nun ein vorteilhaftes Verfahren gefunden zur Abtrennung von Wasser aus einem Wasser und Alkohole und/oder Carbonsäuren und/oder Carbonsäureester enthaltenden Gemisch durch Pervaporation oder Dämpfepermeation, indem das Gemisch mit einer Seite einer Membran in Berührung gebracht wird und das Wasser enthaltende Permeat als Dampf von der anderen Seite der Membran entfernt wird, welches dadurch gekennzeichnet ist, daß man eine Membran verwendet, die durch Plasmapolymerisation erhalten worden ist.

Mit dem neuen Verfahren läßt sich Wasser aus einem Wasser und Alkohole und/oder Carbonsäuren und/oder Carbonsäureester enthaltenden Gemisch, insbesondere das Reaktionswasser aus dem bei der Carbonsäureesterherstellung in Gegenwart von sauren Katalysatoren erhaltenen Reaktionsgemisch, wegen der hohen Wasserselektivität und Permeabilität der verwendeten Membranen und ihrer gleichzeitigen Temperatur-, Säure- und Lösungsmittelbeständigkeit vorteilhaft und wirtschaftlich abtrennen.

Das erfindungsgemäße Verfahren wird mit besonderem Vorteil für die Abtrennung von Reaktionswasser aus dem bei der Herstellung von Carbonsäureestern durch Umsetzung von Alkoholen mit Carbonsäuren in Gegenwart von sauren Katalysatoren erhaltenen Reaktionsgemisch angewendet.

Als Alkohole kommen für die Carbonsäureester-Herstellung im allgemeinen ein- oder zweiwertige, vorzugsweise einwertige aliphatische Alkohole mit 1 bis 12 C-Atomen, vorzugsweise 1 bis 10 C-Atomen, insbesondere 1 bis 8 C-Atomen in Betracht. Geeignete Alkohole sind beispielsweise Methanol, Ethanol, n-Propanol, Isopropanol, n-Butanol, Isobutanol, die Pentylalkohole, die Hexylalkohole, 2-Ethylhexylakohol, Methylglykol, Ethylglykol, Butylglykol, Glykol, Diethylenglykol, Propylenglykol, 1,4-Butandiol, 1,5-Pentandiol.

Als Carbonsäuren kommen für die Carbonsäureester-Herstellung im allgemeinen Mono- oder Dicarbonsäuren, vorzugsweise Monocarbonsäuren, mit 1 bis 10 C-Atomen, vorzugsweise 1 bis 6 C-Atomen, insbesondere 1 bis 4 C-Atomen in Betracht. Geeignete Carbonsäuren sind z.B. Ameisensäure, Essigsäure, Propionsäure, Buttersäure.

Als saure Katalysatoren kommen für die Carbonsäureester-Herstellung im allgemeinen Mineralsäuren wie Schwefelsäure, Phosphorsäure, organische Sulfonsäuren wie Benzolsulfonsäure, p-Toluolsulfonsäure sowie vorzugsweise Ionenaustauscher in der Wasserstoffform, z.B. sulfonierte Polystyrol-Harze wie Kern-sulfonierte vernetzte Styrol-Divinylbenzol-Copolymerisate in Betracht.

Im allgemeinen werden für die Veresterung Temperaturen von 20°C bis 150°C, vorzugsweise 40°C bis 120°C angewandt.

Beispiele für Carbonsäureester, die nach dem erfindungsgemäßen Verfahren erhalten werden, sind n-Propylacetat, iso-Butylacetat, n-Pentylacetat, 2-Methylbutyl-1-acetat, 3-Methylbutyl-1-acetat, 1,5-Pentylendiacetat, 2-Ethylhexylacetat, n-Propylpropionat, n-Pentylpropionat, 2-Methylbutyl-1-propionat, 3-Methylbutyl-1-propionat, Methylglykolacetat, Ethylglykolacetat, Butylglykolacetat, Butyldiglykolacetat, Propylenglykoldiacetat.

Die Abtrennung des Wassers aus dem Wasser und Alkohole und/oder Carbonsäuren und/oder Carbonsäureester enthaltenden Gemisch, beispielsweise aus dem Reaktionsgemisch der Veresterungsreaktion, erfolgt durch Pervaporation oder Dämpfepermeation, indem das Gemisch mit einer Seite einer Membran in Berührung gebracht wird und das Wasser enthaltende Permeat als Dampf von der anderen Seite der Membran entfernt wird.

Geeignete Vorrichtungen für die Trennung von Gemischen nach dem erfindungsgemäßen Verfahren durch Pervaporation bzw. Dämpfepermeation sind z.B. in der Weise aufgebaut, daß sie eine Rohgemischkammer für das aufzutrennende Gemisch, eine Permeatkammer für das abgetrennte Permeat und eine zwischen diesen angeordnete Membran enthalten sowie mit zumindest einem mit der Rohgemischkammer verbundenen Zulaufkanal und zumindest einem mit der Permeatkammer verbundenen Ablaufkanal versehen sind (vgl. z.B. DE-A1-3 529 175). Die Rohgemischkammer enthält zweckmäßig noch einen Ablaufkanal für den Teil des Gemisches (Retentat), der von der Membran zurückgehalten wird.

Die erfindungsgemäße Pervaporation bzw. Dämpfepermeation wird zweckmäßig in der Weise durchgeführt, daß die Konzentration des Wassers im Permeat höher ist als die Konzentration des Wassers im eingesetzten Gemisch und die Konzentrationen der organischen Komponenten im Permeat niedriger sind als im eingesetzten Gemisch.

Das dampfförmig auf der Permeatseite der Membran erhaltene Permeat kann als solches, d.h. dampfförmig, weitergeleitet werden. Es kann jedoch auch vorteilhaft sein, das erhaltene dampfförmige Permeat anschließend zu kondensieren und flüssig weiterzuleiten.

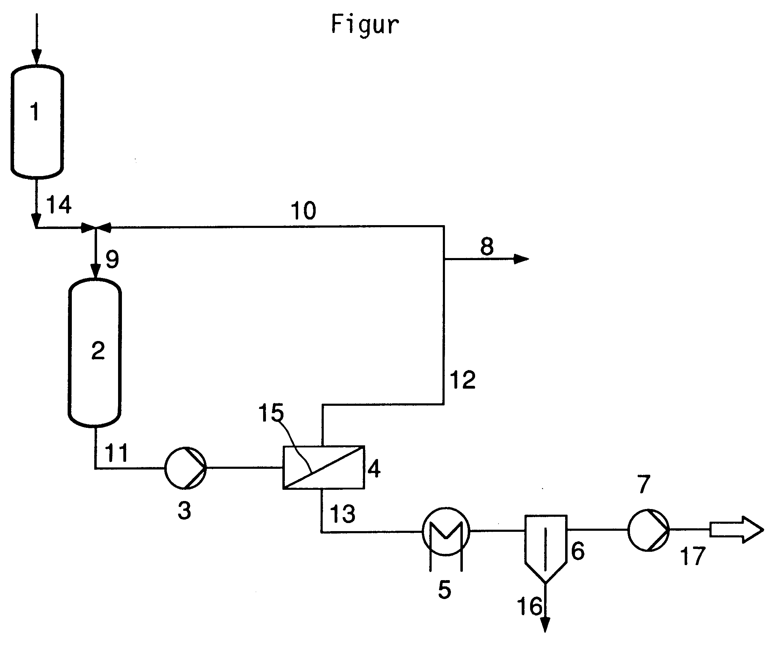

Nachfolgend werden weitere Einzelheiten der Erfindung anhand eines Ausführungsbeispiels für eine Veresterungsreaktion, deren Verfahrensablauf in der Figur schematisch dargestellt ist, erläutert.

Gemäß der Figur werden die Reaktionskomponenten Alkohol und Carbonsäure im Behälter 1 vorgemischt, die Mischung über Leitung 14 abgezogen und mit dem Rückführstrom 10 zu 9 vereinigt und dem Reaktor 2 zugeführt. Der Reaktoraustrag, der den gebildeten Carbonsäureester, Reaktionswasser und gegebenenfalls nicht umgesetzten Alkohol und/oder nicht umgesetzte Carbonsäure enthält, wird mit der Pumpe 3 durch die Vorrichtung 4 zur Abtrennung des Wasser aus dem Reaktionsgemisch durch Pervaporation bzw. Dämpfepermeation ("Pervaporationsvorrichtung") gepumpt. In Vorrichtung 4 wird das Reaktionsgemisch an der Membran 15 in das Reaktionswasser enthaltende Permeat 13 und das Carbonsäureester und gegebenenfalls nicht umgesetzten Alkohol bzw. nicht umgesetzte Carbonsäure enthaltende Retentat 12 aufgeteilt. Der Retentatstrom 12 wird seinerseits in den Carbonsäureester-Produktstrom 8 und den Rückführstrom 10 aufgeteilt. Das dampfförmig abgezogene Permeat 13 wird im Wärmetauscher 5 kondensiert und in Behälter 6 gesammelt, aus dem das Kondensat über Leitung 16 flüssig abgezogen wird. Nichtkondensierte Bestandteile des Permeats werden mit Vakuumpumpe 7 über Leitung 17 ausgetragen.

Es ist ein wesentliches Merkmal des erfindungsgemäßen Verfahrens, daß für die Pervaporation bzw. Dämpfepermeation Membranen verwendet werden, die durch Plasmapolymerisation (vgl. z.B. H. Suhr, Houben-Weyl 4/5 b, Seiten 1559-1591; J. of Macromolecular Science Chemistry, A 10 (3), Seiten 367-368 (1976) hergestellt worden sind. Vorzugsweise werden als Membranen Kompositmembranen verwendet, die aus einer dichten porenfreien Schicht auf einem Trägermaterial bestehen, wobei die dichte porenfreie Schicht durch Plasmapolymerisation auf dem Trägermaterial aufgetragen wird. Die Schichtbildung durch Plasmapolymerisation erfolgt zweckmäßig in der Weise, daß zweckmäßig im Vakuum durch Einwirkung eines Plasmas, das im allgemeinen durch Glimmentladung erzeugt wird, auf ein Arbeitsgas reaktive Spezies erzeugt werden und durch Reaktion der reaktiven Spezies miteinander und/oder mit den Ausgangskomponenten des Arbeitsgases dichte hochvernetzte Polymerschichten auf dem Trägermaterial gebildet werden.

Als Trägermaterial werden zweckmäßig poröse Trägermaterialien, vorzugsweise asymmetrische poröse Trägermaterialien, d.h. poröse Trägermaterialien, die auf der Vorder- und Rückseite Poren mit unterschiedlichem mittlerem Porendurchmesser aufweisen, verwendet. Geeignete Trägermaterialien sind z.B. auf der Basis von Polyacrylnitril aufgebaut. Vorzugsweise werden Ultrafiltrationsmembranen als poröse Trägermaterialien verwendet. Mit besonderem Vorteil werden Kompositmembranen verwendet, die aus einer durch Plasmapolymerisation erzeugten dichten porenfreien Schicht auf einem asymmetrischen porösen Trägermaterial bestehen, wobei die Seite des asymmetrischen porösen Trägermaterials mit den kleineren Poren zur dichten porenfreien Schicht weist.

Als Arbeitsgase für die Plasmapolymerisation werden im allgemeinen unter den angewandten Druckbedingungen gas- oder dampfförmige organische Verbindungen verwendet. Geeignete organische Verbindungen sind z.B. gesättigte und ungesättigte gegebenenfalls substituierte Kohlenwasserstoffe mit im allgemeinen 1 bis 18 C-Atomen, vorzugsweise 1 bis 15 C-Atomen, insbesondere 1 bis 10 C-Atomen. Beispielsweise seien genannt aliphatische Kohlenwasserstoffe wie Methan, Ethan, Propan, Butane, Hexane, Ethylen, Propylen. Butylen, Butadien, Acetylen, Halogenkohlenwasserstoffe wie Chlor-, Brom- und Fluorkohlenwasserstoffe, aromatische Kohlenwasserstoffe wie Benzol, Toluol, Xylol, Ethylbenzol, Monomeren wie Vinylchlorid, Acrylnitril.

Vorzugsweise wird die Plasmapolymerisation mit einem Gasgemisch durchgeführt, das eine gas- oder dampfförmige organische Verbindung und ein anorganisches Gas enthält. Dabei wird mit besonderem Vorteil ein solches Gasgemisch verwendet, in dem wenigstens eine Komponente, vorzugsweise das anorganische Gas, Stickstoff enthält. Als stickstoffhaltige anorganische Gase werden im allgemeinen N₂, NH₃ oder ein Oxid des Stickstoffs wie N₂O, NO, NO₂, N₂O₃, N₂O₅ verwendet.

Die Plasmapolymerisation wird z.B. in der Weise durchgeführt, daß der Reaktor, zweckmäßig ein Entladungsgefäß, in dem sich das poröse Trägermaterial befindet, mit dem polymerisierenden Gasgemisch geflutet und dann eine Glimmentladung gezündet wird. Diese Glimmentladung führt zur Abscheidung einer dichten Plasmapolymerschicht auf dem porösen Trägermaterial. Nach beendeter Plasmapolymerisation wird die Membran dem Reaktor entnommen.

Zweckmäßig wird die Glimmentladung mit einer Wechselfrequenz betrieben. Geeignete Glimmentladungen können im Niederfrequenz- und Hochfrequenzbereich erzeugt werden, z.B. im Wechselstrombereich, im kHz-Bereich, im MHz-Bereich, im Mikrowellenbereich. Im allgemeinen werden dabei im Entladungsgefäß Gasflüsse zwischen 0 und 1000 ml(NTP)/min, die elektrische Leistung zwischen 0,001 und 5 W/cm² und der Druck zwischen 1·10⁻⁶ und 1·10³ mbar, vorzugsweise zwischen 1·10⁻³ und 1·10¹ mbar eingestellt. Im allgemeinen beträgt die Behandlungszeit 0,1 s bis 120 min, vorzugsweise 1 s bis 60 min, insbesondere 5 s bis 30 min. Es kann vorteilhaft sein, das Entladungsgefäß mit innenliegenden Elektroden zu betreiben. Weiter kann es dabei vorteilhaft sein, das poröse Trägermaterial mit einer Elektrode in Kontakt zu bringen. Dies kann auch dazu benutzt werden, das poröse Trägermaterial, z.B. von einer Rolle, für eine kontinuierliche Beschichtung als Band über die Elektrode zu bewegen.

Die erfindungsgemäß erhaltenen Membranen zeichnen sich durch eine hohe Permeabilität und Selektivität für Wasser aus. Auch bei längerem Betrieb erlitt das Membranmaterial praktisch keine Einbuße in bezug auf die Permeabilität und Selektivität.

Die folgenden Beispiele veranschaulichen die Erfindung.
A) Herstellung der Komposit-Pervaporationsmembranen für die Wasserabtrennung bei der Veresterung
   a) Allgemeine Herstellvorschrift
      Zur Herstellung der Komposit-Pervaporationsmembranen werden Ultrafiltrationsmembranen der Fa. GFT mbH, Neunkirchen/Heinitz als Trägermaterial ("UF-Trägermembran") verwendet. Diese Membranen werden durch Phaseninversion hergestellt. Sie haben Molekularausschlußgrenzen von > 5000 Dalton und bestehen aus Polyacrylnitril.
      Die Abscheidung der porenfreien Trennschicht durch Plasmapolymerisation wird in einem mit zwei Elektroden ausgestatteten Vakuumgefäß durchgeführt. Eine der beiden Elektroden ist mit einem Hochfrequenz-Hochspannungs-Generator verbunden. Die zweite Elektrode ist geerdet. Zur Herstellung der Kompositmembran wird das Trägermaterial auf einer der beiden Elektroden befestigt. Anschließend wird das Gefäß evakuiert (p<1·10⁻³ mbar) und mit dem Prozessgas geflutet. Nach der Abscheidung der Trennschicht wird das Gefäß erneut evakuiert und dann belüftet und die erhaltene Kompositmembran dem Gefäß entnommen.
   b) Herstell-Beispiele
      Beispiel 1
      Man verfährt gemäß der allgemeinen Herstellvorschrift, wobei der Reaktor mit einem Gemisch aus Ammoniak und p-Xylol geflutet wird und der NH₃-Fluß 22 ml(NTP)/min und der p-Xylol-Fluß 14 ml/min beträgt. Der Druck im Reaktor beträgt 0,4 mbar. Die Entladung wird mit einer Leistung von 150 W betrieben und für 5 Minuten aufrechterhalten.
      Beispiel 2
      Man verfährt gemäß der allgemeinen Herstellvorschrift, wobei der Reaktor mit einem Gemisch aus Stickstoff und Ethylen geflutet wird und der N₂-Fluß 30 ml(NTP)/min und der Ethylen-Fluß 50 ml(NTP)/min beträgt. Der Druck im Reaktor beträgt 0,4 mbar. Die Entladung wird mit einer Leistung von 100 W betrieben und für 6 Minuten aufrechterhalten.
      Beispiel 3
      Man verfährt gemäß der allgemeinen Herstellvorschrift, wobei der Reaktor mit einem Gemisch aus Ammoniak, Kohlendioxid und Ethylen geflutet wird und der NH₃-Fluß 25 ml(NTP)/min, der CO₂-Fluß 25 ml(NTP/min) und der C₂H₄-Fluß 40 ml(NTP)/min beträgt. Der Druck im Reaktor beträgt 0,4 mbar. Die Entladung wird mit einer Leistung von 150 W betrieben und für 10 Minuten aufrechterhalten.
      Beispiel 4
      Man verfährt gemäß der allgemeinen Herstellvorschrift, wobei der Reaktor mit einem Gemisch aus Ammoniak und Ethylen geflutet wird und der NH₃-Fluß 50 ml(NTP)/min und der C₂H₄-Fluß 50 ml(NTP)/min beträgt. Der Druck im Reaktor beträgt 0,8 mbar. Die Entladung wird mit einer Leistung von 400 W betrieben und für 5 Minuten aufrechterhalten.
B) Wasserabtrennung in einer Pervaporationsvorrichtung aus bei der Umsetzung von n-Butanol und Essigsäure zu n-Butylacetat erhaltenen Gemischen
   Beispiel 5
   Bei der Zuführung eines Gemisches aus 9 Gew.-% Wasser, 15 Gew.-% Essigsäure, 18 Gew.-% n-Butanol und 58 Gew.-% n-Butylacetat zu einer mit einer Kompositmembran gemäß Beispiel 1 ausgestatteten Pervaporationsvorrichtung liefert diese Membran bei einem Gesamtfluß von 0,6 kg/m²h ein Permeatgemisch mit 98,5 Gew.-% Wasser und 1,5 Gew.-% organischen Bestandteilen.
   Beispiel 6
   Bei der Zuführung eines Gemisches aus 1,5 Gew.-% Wasser, 5 Gew.-% Essigsäure, 6 Gew.-% n-Butanol und 87,5 Gew.-% n-Butylacetat zu einer mit einer Kompositmembran gemäß Beispiel 1 ausgestatteten Pervaporationsvorrichtung liefert dieses Membran bei einem Gesamtfluß von 0.1 kg/m²h ein Permeatgemisch mit 99 Gew.-% Wasser und 1 Gew.-% organische Bestandteilen.
   Die Beispiele 5 und 6 zeigen die hohe Wasserselektivität der Membran, die auch bei unterschiedlicher Zusammensetzung des Einsatzgemisches für die Pervaporation Permeatströme mit einem Wassergehalt von jeweils mehr als 98 Gew.-% ergibt bei gleichzeitig hoher Wasserpermeabilität der Membran.
   Beispiel 7
   Man verfährt wie in Beispiel 6 beschrieben, wobei jedoch als Kompositmembran die gemäß Beispiel 2 hergestellte Membran verwendet wird, die bei einem Gesamtfluß von 0,015 kg/m²h ein Permeatgemisch mit 83 Gew.-% Wasser und 17 Gew.-% organischen Bestandteilen liefert.
   Beispiel 8
   Man verfährt wie in Beispiel 6 beschrieben, wobei jedoch als Kompositmembran die gemäß Beispiel 3 hergestellte Membran verwendet wird, die bei einem Gesamtfluß von 0,3 kg/m²h ein Permeatgemisch mit 91 Gew.-% Wasser und 9 Gew.-% organischen Bestandteilen liefert.
   Beispiel 9
   Man verfährt wie in Beispiel 5 beschrieben, wobei jedoch als Kompositmembran die gemäß Beispiel 4 hergestellte Membran verwendet wird, die bei einem Gesamtfluß von 3,5 kg/m²h ein Permeatgemisch mit 97,5 Gew.-% Wasser und 2,5 Gew.-% organischen Bestandteilen liefert.
   Beispiel 10
   Man verfährt wie in Beispiel 6 beschrieben, wobei jedoch als Kompositmembran die gemäß Beispiel 4 hergestellte Membran verwendet wird, die bei einem Gesamtfluß von 0,7 kg/m²h ein Permeatgemisch mit 99 Gew.-% Wasser und 1 Gew.-% organischen Bestandteilen liefert.
   Beispiel 11
   In der Apparatur gemäß der Figur, in der in der Pervaporationsvorrichtung 4 die gemäß Beispiel 1 erhaltene Membran als Membran 15 verwendet wird, wird durch Veresterung von n-Butanol mit Essigsäure in Gegenwart von einem Ionenaustauscher als saurer Katalysator n-Butylacetat hergestellt. Der aus dem Veresterungsreaktor 2 erhaltene Produktstrom enthält den Ester n-Butylacetat in einer Konzentration von 62 Gew.-%. Durch selektive Wasserabtrennung in der nachgeschalteten Pervaporationsstufe in Vorrichtung 4 wird ein Produktstrom 8 mit einer Esterkonzentration von 88 Gew.-% erhalten.

## Patentansprüche

1. Verfahren zur Abtrennung von Wasser aus einem Wasser und Alkohole und/oder Carbonsäuren und/oder Carbonsäureester enthaltenden Gemisch durch Pervaporation oder Dämpfepermeation, indem das Gemisch mit einer Seite einer Membran in Berührung gebracht wird und das Wasser enthaltende Permeat als Dampf von der anderen Seite der Membran entfernt wird, dadurch gekennzeichnet, daß man eine Membran verwendet, die durch Plasmapolymerisation erhalten worden ist.

2. Verfahren nach Anspruch 1, dadurch gekennzeichnet, daß man es für die Herstellung von Carbonsäureestern durch Umsetzung von Alkoholen mit Carbonsäuren in Gegenwart von sauren Katalysatoren anwendet und dabei das gebildete Reaktionswasser aus dem erhaltenen Reaktionsgemisch als Permeat abtrennt.

3. Verfahren nach Anspruch 2, dadurch gekennzeichnet, daß als saure Katalysatoren Mineralsäuren verwendet werden.

4. Verfahren nach Anspruch 2, dadurch gekennzeichnet, daß als saure Katalyatoren Ionenaustauscher verwendet werden.

5. Verfahren nach Anspruch 1 bis 4, dadurch gekennzeichnet, daß für die Abtrennung des Wassers eine Kompositmembran bestehend aus einer dichten porenfreien Schicht und einem asymmetrischen porösen Trägermaterial verwendet wird, wobei die Seite des asymmetrischen porösen Trägermaterials mit den kleineren Poren zur dichten porenfreien Schicht weist und die dichte porenfreie Schicht durch Plasmapolymerisation erhalten worden ist.

6. Verfahren nach den Ansprüchen 1 bis 5, dadurch gekennzeichnet, daß die Plasmapolymerisation durch Glimmentladung in einem Gasgemisch, das eine gas- oder dampfförmige organische Verbindung und ein anorganisches Gas enthält, durchgeführt wird.

7. Verfahren nach Anspruch 6, dadurch gekennzeichnet, daß die Plasmapolymerisation in einem Gasgemisch durchgeführt wird, in dem wenigstens eine Komponente Stickstoff enthält.

8. Verfahren nach Anspruch 7, dadurch gekennzeichnet, daß das Gasgemisch ein stickstoffhaltiges anorganisches Gas enthält.

9. Verfahren nach Anspruch 8, dadurch gekennzeichnet, daß als stickstoffhaltiges anorganisches Gas N₂, NH₃ oder ein Oxid des Stickstoffs oder ein Gemisch von zwei oder mehreren dieser Gase verwendet wird.

## Claims

1. A process for removing water from a mixture of water and an alcohol, a carboxylic acid or a carboxylic ester by pervaporation or vapor permeation by bringing the mixture into contact with one side of a membrane and removing the water-containing permeate in vapor form from the other side of the membrane, which comprises using a membrane obtained by plasma polymerization.

2. A process as claimed in claim 1, wherein the mixture is a reaction mixture from the preparation of a carboxylic ester by reacting an alcohol with a carboxylic acid in the presence of an acidic catalyst and the water removed as permeate is the water of reaction.

3. A process as claimed in claim 2, wherein the acidic catalyst used is a mineral acid.

4. A process as claimed in claim 2, wherein the acidic catalyst used is an ion exchange material.

5. A process as claimed in claim 1 or 2 or 3 or 4, wherein the membrane used for removing the water is a composite membrane comprising a dense nonporous layer and an asymmetrical porous support material, the side of the asymmetrical porous support material facing with the smaller pores toward the dense nonporous layer and the dense nonporous layer having been obtained by plasma polymerization.

6. A process as claimed in claim 1 or 2 or 3 or 4 or 5, wherein the plasma polymerization is performed by glow discharge in a gas mixture comprising a gaseous or vaporous organic compound and an inorganic gas.

7. A process as claimed in claim 6, wherein the plasma polymerization is performed in a gas mixture in which at least one component is nitrogen.

8. A process as claimed in claim 7, wherein the gas mixture contains a nitrogen-containing inorganic gas.

9. A process as claimed in claim 8, wherein the nitrogen-containing inorganic gas used is N₂, NH₃ or an oxide of nitrogen or a mixture of two or more of these gases.

## Revendications

1. Procédé de séparation de l'eau d'un mélange contenant de l'eau et des alcools et/ou des acides carboxyliques et/ou des esters d'acides carboxyliques par pervaporation ou perméation de vapeurs, conformément auquel on met le mélange en contact avec un côté d'une membrane et on élimine le perméat contenant de l'eau sous forme de vapeur de l'autre côté de la membrane, caractérisé en ce que l'on utilise une membrane obtenue par polymérisation au plasma.

2. Procédé suivant la revendication 1, caractérisé en ce qu'on le met en oeuvre pour la préparation d'esters d'acides carboxyliques par la réaction d'alcools avec des acides carboxyliques en présence de catalyseurs acides et, au cours de cette réaction, on sépare l'eau de réaction formée du mélange de réaction obtenu sous forme de perméat.

3. Procédé suivant la revendication 2, caractérisé en ce que l'on utilise des acides minéraux à titre de catalyseurs acides.

4. Procédé suivant la revendication 2, caractérisé en ce que l'on utilise des échangeurs d'ions à titre de catalyseurs.

5. Procédé suivant l'une quelconque des revendications 1 à 4, caractérisé en ce que, pour la séparation de l'eau, on utilise une membrane composite constituée d'une couche étanche dépourvue de pores et d'une matière de support poreuse asymétrique, où le côté de la matière de support poreuse asymétrique avec les pores les plus petits fait face à la couche étanche dépourvue de pores et la couche étanche dépourvue de pores est obtenue par polymérisation au plasma.

6. Procédé suivant l'une quelconque des revendications 1 à 5, caractérisé en ce que l'on entreprend la polymérisation au plasma par décharge électrique à faible luminescence dans un mélange de gaz qui contient un composé organique sous forme de gaz ou de vapeur et un gaz inorganique.

7. Procédé suivant la revendication 6, caractérisé en ce que l'on entreprend la polymérisation au plasma dans un mélange de gaz, dans lequel au moins l'un des composants contient de l'azote.

8. Procédé suivant la revendication 7, caractérisé en ce que le mélange de gaz contient un gaz inorganique contenant de l'azote.

9. Procédé suivant la revendication 8, caractérisé en ce que l'on utilise de l'azote, de l'ammoniac ou un oxyde de l'azote ou un mélange de deux ou de plus de deux de ces gaz à titre de gaz inorganique contenant de l'azote.
